# EUROPEAN PATENT SPECIFICATION

(11) **EP 3 851 526 B1**
(45) Date of publication and mention of the grant of the patent: **26.11.2025**
(21) Application number: 19847258.1
(22) Date of filing: 08.08.2019
(51) Int. Cl.: C12N 13/00, C12N 5/0735, C12N 5/0775

(54) **USE OF NANOSECOND PULSED ELECTRIC FIELD IN IMPROVING CELL STEMNESS**
VERWENDUNG VON NANOSEKUNDENGEPULSTEM ELEKTRISCHEM FELD ZUR VERBESSERUNG DER STAMMZELLFÄHIGKEIT VON ZELLEN
UTILISATION D'UN CHAMP ÉLECTRIQUE PULSÉ EN NANOSECONDE DANS L'AMÉLIORATION DU CARACTÈRE SOUCHE D'UNE CELLULE

(30) Priority: 10.08.2018 CN 201810909901
(43) Date of publication of application: 21.07.2021
(73) Proprietor: Peking University, Beijing 100871 (CN)
(72) Inventor: GE, Zigang, Beijing 100871 (CN); NING, Tong, Beijing 100871 (CN); ZHANG, Jue, Beijing 100871 (CN); CHEN, Jiaqing, Beijing 100871 (CN); GUO, Jinsong, Beijing 100871 (CN); LI, Kejia, Beijing 100871 (CN)
(74) Representative: Schnappauf, Georg
(86) International application number: PCT/CN2019/099746
(87) International publication number: WO 2020/030037

(56) References cited:
- WO-A2-2007/100727
- NING T ET AL: "Nanosecond pulsed electric fields enhance chondrogenesis of mesenchymal stem cells (MSCS) partially via phosphorylation of P38-MAPK", OSTEOARTHRITIS AND CARTILAGE, ELSEVIER, AMSTERDAM, NL, vol. 24, 284, 30 April 2016 (2016-04-30), pages S170, XP029466990, ISSN: 1063-4584, DOI: 10.1016/J.JOCA.2016.01.333
- PETRELLA ROSS A ET AL: "Non-Contact Picosecond Pulsed Electric Fields Up Regulate SOX2 Gene Expression in Mesenchymal Stem Cells", 2018 IEEE INTERNATIONAL MICROWAVE BIOMEDICAL CONFERENCE (IMBIOC), IEEE, 14 June 2018 (2018-06-14), pages 100 - 102, XP033382991, DOI: 10.1109/IMBIOC.2018.8428906
- PETRELLA ROSS AARON: "Non-Invasive Picosecond Pulse System for Electrostimulation", DOCTOR OF PHILOSOPHY (PHD) - OLD DOMINION UNIVERSITY LIBRARIES, 1 May 2018 (2018-05-01), pages 1 - 124, XP055896271, Retrieved from the Internet <URL:https://digitalcommons.odu.edu/cgi/viewcontent.cgi?article=1029&context=ece_etds> [retrieved on 20220301], DOI: 10.25777/ystf-ry94
- ALLEN SUMMER E: "Healing the Burn: Advances in Burn Treatment Technology Aim to Save Lives, Lessen Pain and Scarring", IEEE PULSE, IEEE, USA, vol. 7, no. 4, 1 July 2016 (2016-07-01), pages 24 - 29, XP011616695, ISSN: 2154-2287, [retrieved on 20160712], DOI: 10.1109/MPUL.2016.2563784
- TONG NING ET AL: "Nanosecond pulsed electric fields enhanced chondrogenic potential of mesenchymal stem cells via JNK/CREB-STAT3 signaling pathway", STEM CELL RESEARCH & THERAPY, vol. 10, no. 1, 284, 24 January 2019 (2019-01-24), XP055753151, DOI: 10.1186/s13287-019-1133-0
- KEJIA LI ET AL: "Nanosecond pulsed electric fields enhance mesenchymal stem cells differentiation via DNMT1-regulated OCT4/NANOG gene expression", STEM CELL RESEARCH & THERAPY, vol. 11, no. 1, 22 July 2020 (2020-07-22), XP055752934, DOI: 10.1186/s13287-020-01821-5
- XIONG JIAN-PING, HE PING,LI ZHAO-DONG,LIU LI-MIN,XIE TENG-FEI,SHU MIAN: "Effect on DNA Methylation of Guodao 6 by Strong Electric Field Radiation at Atmospheric Pressure", PROGRESS IN MODERN BIOMEDICINE, vol. 13, no. 6, 28 February 2013 (2013-02-28), pages 1066 - 1070+1099, XP055783821, ISSN: 1673-6273, DOI: 10.13241/j.cnki.pmb.2013.06.004
- NING T; GE Z; LIN J: "284 Nanosecond pulsed electric fields enhance chondrogenesis of mesenchymal stem cells (MSCS) partially via phosphorylation of P38-MAPK", OSTEOARTHRITIS AND CARTILAGE, vol. 24, 30 April 2016 (2016-04-30), pages S170, XP029466990, ISSN: 1063-4584, DOI: 10.1016/j.joca.2016.01.333
- LI-YI SUN; DEAN-KUO HSIEH; TZAI-CHIU YU; HSIEN-TAI CHIU; SHENG-FEN LU; GENG-HONG LUO; TOM K KUO; OSCAR K LEE; TZYY-WEN CHIOU: "Effect of Pulsed Electromagnetic Field on the Proliferation and Differentiation Potential of Human Bone Marrow Mesenchymal Stem Cells", BIOELECTROMAGNETICS, vol. 30, no. 4, 1 May 2009 (2009-05-01), pages 251 - 260, XP055228003, ISSN: 0197-8462, DOI: 10.1002/bem.20472
- THRIVIKRAMAN GREESHMA; BODA SUNIL KUMAR; BASU BIKRAMJIT: "Unraveling the mechanistic effects of electric field stimulation towards directing stem cell fate and function: A tissue engineering perspective", BIOMATERIALS, vol. 150, 3 October 2017 (2017-10-03), pages 60 - 86, XP085246321, ISSN: 0142-9612, DOI: 10.1016/j.biomaterials.2017.10.003
- ALEKSANDRA MAZIARZ; KOCAN BEATA; BESTER MARIUSZ; BUDZIK SYLWIA; CHOLEWA MARIAN; OCHIYA TAKAHIRO; BANAS AGNIESZKA: "How electromagnetic fields can influence adult stem cells: positive and negative impacts", STEM CELL RESEARCH & THERAPY, vol. 7, 54, 1 December 2016 (2016-12-01), pages 1 - 12, XP055685188, DOI: 10.1186/s13287-016-0312-5

## Description

### CROSS REFERENCE TO RELATED APPLICATIONS

This application claims the priority to Chinese Patent Application No. 201810909901.0, titled "USE OF NANOSECOND PULSED ELECTRIC FIELD IN IMPROVING CELL STEMNESS", filed on August 10, 2018 with the China National Intellectual Property Administration.

### FIELD

The present disclosure relates to the technology of using pulsed electric fields for biological treatment, and particularly relates to the technique of using nanosecond pulsed electric fields to treat cells, in particular stem cells.

### BACKGROUND

Differentiation induction of stem cells is an important research direction in regenerative medicine. The induction efficiency of stem cells needs to be improved to achieve better clinical effects. In order to improve the efficiency of differentiation induction of stem cells, a variety of stimulations has been applied, such as physical stimulations, chemical molecules, and cytokines. Electrical signals, as one of the very important physical stimulations, can facilitate differentiation of stem cells, such as neuronal differentiation, osteogenic differentiation and cardiomyocyte differentiation, by regulating the related signaling pathways, such as calcium ion level and ion channels in cells. However, the biological effect of the electrical signals is weak, thus the electric field need to be applied for a long time ranging from several tens of minutes to several days to take action, which is not favorable to clinical applications.

Nanosecond pulsed electric fields (nsPEFs) are an advanced physical technology developed in recent years. The pulse width, field strength and instantaneous power of nsPEFs can be accurately controlled, so compared with the conventional electric fields, it gives stronger biological effect in a shorter action time. However, it is still desired to explore more applications of nanosecond pulsed electric fields in this field.

Ning T, et al. (Osteoarthritis and Cartilage 24 (2016): S170) disclose that nanosecond pulsed electric fields enhance chondrogenesis of mesenchymal stem cells partially via phosphorylation of P38-MAPK. Petrella RA, et al. (IEEE International Microwave Biomedical Conference (IMBioC) (2018): 100-102) disclose that when applying picosecond pulsed electric fields to MSCs *in vitro,* the gene expression of SOX2 was up-regulated. Petrella RA (Doctor of Philosophy - Old Dominion University Libraries (2018): 1-124) discloses the stimulation of neural stem cells, and in a minor part MSCs, by psPEF. Allen Summer E (IEEE Pulse 7 (2016): 24-29) discloses the use of PEFs in the treatment of burns, and mentions their ability to redirect the healing process and reduce scar formation.

### SUMMARY

The present invention is defined by the appended claims.

In the research, the inventors found that the conventional electric fields mostly have a field strength lower than "hundreds of V/cm" and a pulse width higher than microseconds or milliseconds, thereby they only can act at the outside of cell membranes, while nanosecond pulsed electric fields have a pulse width that can reach the nanosecond (ns) level and a field strength that can reach kV/cm level. In additions, nsPEFs can act on organelles within cell membranes. Furthermore, the inventors unexpectedly found that applying nsPEFs on stem cells can significantly enhance the stemness of the stem cells and further enhance its capability to respond to induction, thereby this invention is accomplished.

Described herein is a use of an electric field in enhancing stemness of a cell, increasing the expression of stemness-related genes in a cell, reducing the expression of methylation-related genes in a cell and/or reducing the methylation level in a cell. For example, the cell is a stem cell, such as a pluripotent stem cell, an unipotent stem cell, an embryonic stem cell, an adult stem cell, an iPS cell or a mesenchymal stem cell.

In a first aspect, the present invention provides an use of an electric field in enhancing stemness of a pluripotent stem cell, an embryonic stem cell or an iPS cell *in vitro,* increasing the expression of stemness-related genes in a pluripotent stem cell, an embryonic stem cell or an iPS cell *in vitro,* reducing the expression of methylation-related genes in a pluripotent stem cell, an embryonic stem cell or an iPS cell *in vitro* and/or reducing the methylation level in a pluripotent stem cell, an embryonic stem cell or an iPS cell *in vitro.*

The electric field is a nanosecond pulsed electric field (nsPEF) having a pulse width in a range of 10-150 ns.

Particularly, the electric field is applied to the cell to give an electric shock to the cell.

The cell is a pluripotent stem cell, an embryonic stem cellor an iPS cell.

In an embodiment, the nanosecond pulsed electric field has a field strength in a range of 1-30 kV/cm.

In an embodiment, the pulse width is in a range of 20-100 ns, 30-80 ns, 40-70 ns, or 50-60 ns. In another embodiment, the pulse width is about 10 ns, 15 ns, 20 ns, 25 ns, 30 ns, 35 ns, 40 ns, 45 ns, 50 ns, 55 ns, 60 ns, 65 ns, 70 ns, 75 ns, 80 ns, 85 ns, 90 ns, 95 ns, 100 ns, 105 ns, 110 ns, 115 ns, 120 ns, 125 ns, 130 ns, 135 ns, 140 ns, 145 ns, or 150 ns.

In another embodiment, the field strength is about 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm, or 30 kV/cm.

In another embodiment, the electric shock is applied 1-1000 times, for example, 10-900 times, 20-800 times, 30-700 times, 40-600 times, 50-500 times, 60-400 times, 70-300 times, 80-200 times, or 90-100 times, and specifically 1, 2, 3, 4, 5, 6, 7, 8, 9, 10, 11, 12, 13, 14, 15, 16, 17, 18, 19, 20, 30, 40, 50, 60, 70, 80, 90 or 100 times. And/or, the electric shock is applied at a frequency of 1-1000 Hz, for example, 10-900 Hz, 20-800 Hz, 30-700 Hz, 40-600 Hz, 50-500 Hz, 60-400 Hz, 70-300 Hz, 80-200 Hz, 90-100 Hz, and specifically about 1, 2, 3, 4, 5, 6, 7, 8, 9, 10, 11, 12, 13, 14, 15, 16, 17, 18, 19, 20, 30, 40, 50, 60, 70, 80, 90, 100 Hz, 200 Hz, 300 Hz, 400 Hz, 500 Hz, 600 Hz, 700 Hz, 800 Hz, 900 Hz, or 1000 Hz.

Also described herein is a method for enhancing stemness of a cell, increasing the expression of stemness-related genes in a cell, reducing the expression of methylation genes and/or reducing the methylation level in a cell, comprising applying an electric field to the cell to give an electric shock to the cell, wherein the electric field can a nanosecond pulsed electric field;

For example, the cell is a stem cell, such as a pluripotent stem cell, an unipotent stem cell, an embryonic stem cell, an adult stem cell, an iPS cell or a mesenchymal stem cell.

In a second aspect, the present invention provides a method for enhancing stemness of a pluripotent stem cell, an embryonic stem cell or an iPS cell *in vitro,* increasing the expression of stemness-related genes in a pluripotent stem cell, an embryonic stem cell or an iPS cell *in vitro,* reducing the expression of methylation genes and/or reducing the methylation level in a pluripotent stem cell, an embryonic stem cell, or an iPS cell *in vitro,* comprising applying an electric field to the cell to give an electric shock to the cell. The electric field is a nanosecond pulsed electric field having a pulse width in a range of 10-150 ns.

In one embodiment, the nanosecond pulsed electric field has a field strength in a range of 1-30 kV/cm.

Preferably, the pulse width is in a range of 20-100 ns, 30-80 ns, 40-70 ns, or 50-60 ns or is, for example, about 10 ns, 15 ns, 20 ns, 25 ns, 30 ns, 35 ns, 40 ns, 45 ns, 50 ns, 55 ns, 60 ns, 65 ns, 70 ns, 75 ns, 80 ns, 85 ns, 90 ns, 95 ns, 100 ns, 105 ns, 110 ns, 115 ns, 120 ns, 125 ns, 130 ns, 135 ns, 140 ns, 145 ns, or 150 ns.

In addition, a preferred field strength is about 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm, or 30 kV/cm.

In another embodiment, the electric shock is applied 1-1000 times, for example, 10-900 times, 20-800 times, 30-700 times, 40-600 times, 50-500 times, 60-400 times, 70-300 times, 80-200 times, 90-100 times, and specifically 1, 2, 3, 4, 5, 6, 7, 8, 9, 10, 11, 12, 13, 14, 15, 16, 17, 18, 19, 20, 30, 40, 50, 60, 70, 80, 90 or 100 times. And/or, the electric shock is applied at a frequency of 1-1000 Hz, for example, 10-900 Hz, 20-800 Hz, 30-700 Hz, 40-600 Hz, 50-500 Hz, 60-400 Hz, 70-300 Hz, 80-200 Hz and 90-100 Hz, and specifically about 1, 2, 3, 4, 5, 6, 7, 8, 9, 10, 11, 12, 13, 14, 15, 16, 17, 18, 19, 20, 30, 40, 50, 60, 70, 80, 90, 100 Hz, 200 Hz, 300 Hz, 400 Hz, 500 Hz, 600 Hz, 700 Hz, 800 Hz, 900 Hz, or 1000 Hz.

In another embodiment, the electric field is applied via an electroporation cuvette.

In another embodiment, the electric field is applied via a conductive film, and preferably, the conductive film is prepared from PLLA (poly-L-lactic acid) and MWCNTs (multi-walled carbon nanotubes) by blend-casting or electrospinning.

In a specific embodiment, the conductive film is prepared by blend-casting, and specifically by: 1) dissolving the poly-L-lactic acid in a solvent (e.g., chloroform) until fully dissolved; 2) dispersing the multi-walled carbon nanotubes (e.g., carboxylated carbon nanotubes) in the same solvent; 3) performing ultra-sonication (e.g., water bath ultrasound) on the multi-walled carbon nanotubes mixture of step 2), and adding into the poly-L-lactic acid solution of step 1), to allow the final concentration of poly-L-lactic acid is 1-50% w/v; 4) after ultra-sonication, casting the resultant on a plate (e.g., glass plate) and standing (preferably standing overnight); 5) peeling off the film formed on the plate; and 6) placing the film into an oven (e.g., vacuum oven) to fully evaporate the solvent (preferably at a temperature of 35°C), and obtain a conductive film.

In another specific embodiment, the polylactic acid has a molecular weight in a range of 10,000-1,000,000 Daltons and an intrinsic viscosity of 1-20 dL/g.

Moreover, described herein is a method for enhancing the capability of a mesenchymal stem cell to differentiate into chondrocyte, osteocyte or adipocyte, comprising applying a nanosecond pulsed electric field to the mesenchymal stem cell to give an electric shock to the mesenchymal stem cell.

Also described herein is a cell generated by the method in the second aspect of the present invention, and a pharmaceutical composition, comprising the cell.

The present disclosure unexpectedly found that nanosecond pulsed electric fields are able to enhance stemness of a stem cell, and the method to enhance stemness is suitable for a various types of stem cells, and the enhancement on stemness of a stem cell will further facilitate its capability of responding to induction and differentiation. Therefore, the technical solutions of the present disclosure are of immeasurable value on improving the activity and differentiation potential of stem cells, and on promoting the stem cells to be used more wildly and effectively in the regenerative medicine.

### BRIEF DESCRIPTION OF DRAWINGS

FIG. 1A is an image of a nanosecond pulsed electric field device, FIG. 1B is a side view of an electroporation cuvette, FIG. 1C is a top view of an electroporation cuvette, FIG. 1D is a schematic drawing of electroporation cuvettes used in the cell experiments and their field strength simulation, and FIG. 1E is a graph showing the effect on the cell survival status under different electric shock conditions;
FIG. 2 is graphs showing the detection results of stemness genes and methylation-related genes in porcine bone marrow mesenchymal stem cells stimulated by nanosecond pulsed electric fields;
FIG. 3 is a graph showing the detection results of the overall methylation level in porcine bone marrow mesenchymal stem cells stimulated by nanosecond pulsed electric fields;
FIG. 4 is graphs showing the results of alcian blue staining (A), GAG semi-quantification (B), and the gene expression of COLII, ACAN and Sox9 (C), after the cells were stimulated by nanosecond pulsed electric fields and differentiated into chondroblasts;
FIG. 5 is a graph showing the results of alizarin red-based semi-quantification, after the cells were stimulated by nanosecond pulsed electric fields and differentiated into osteoblasts;
FIG. 6 is a graph showing the results of oil red O-based semi-quantification, after the cells were stimulated by nanosecond pulsed electric fields and differentiated into adipoblasts;
FIG. 7 is graphs showing the detection results of stemness genes of OCT4, NANOG and SOX2 in human embryonic stem cells stimulated by nanosecond pulsed electric fields;
FIG. 8 is an image of a real conductive film (A) and its scanning electron micrograph (B); and
FIG. 9 is graphs showing the comparison of the effects on the expression of stemness genes after stimulating mesenchymal stem cells via an electroporation cuvette, and that of via a conductive thin film.

### DETAILED DESCRIPTION

The present disclosure can be further understood by reference to the embodiments.

### Definitions

The term "stemness" refers to a capacity of a stem cell to maintain in its undifferentiated state. Stem cells keep a potential capacity to differentiate into one or more cell fate(s) and a potential capacity to be induced to differentiate, while being in continually self-renewal. Stemness of a stem cell can be detected by morphological observation or by detecting stemness genes of the cell. The term "stemness gene", also called as "stemness marker gene" or "stemness-related gene", refers to the genes associated with the status of a stem cell. The expression of such genes is beneficial for maintaining the stemness of a stem cell. The common stemness-related genes include Oct4, Nanog, Sox2 and so on.

Additionally, stemness of a stem cell may also be characterized by a low methylation level in the cell, and the reduction in methylation level can be achieved by regulating down the expression of methylation genes. Therefore, stemness of a stem cell may be further characterized by the low-expression (reduction in expression) of methylation genes in a cell. In the present disclosure, the term "methylation-related gene", also called as "methylation gene", refers to the genes which facilitate the methylation in a cell to increase the methylation level in the cell, such as methyltransferase genes, and more specifically Dnmt1.

The term "electric shock" used herein, is also called as "electrical stimulation" or "electrical field stimulation", refers to apply an electric field (or electric pulses) having a certain pulse width and field strength to a subject, such as cells (including stem cells), for a set times at a set frequency. In one embodiment, the electric shock is applied via a specialized device. In one particular embodiment, the electric shock is applied via an electroporation cuvette. In another specific embodiment, the electric shock is applied via a conductive film (or called as a conductive thin film).

The conductive film may be prepared by using any materials, structures and methods known in the prior art, as long as it is suitable for applying electric fields. For example, a nanofiber and a preparation method disclosed by Zhu S, Jing W, Hu X, Huang Z, Cai Q, Ao Y, Yang X. 2017 (Time-dependent effect of electrical stimulation on osteogenic differentiation of bone mesenchymal stromal cells cultured on conductive nanofibers, J Biomed Mater Res Part A 2017:105A:3369-3383).

In one embodiment, the conductive film is prepared from poly-L-lactic acid (PLLA) and multi-walled carbon nanotubes (MWCNTs) by blend-casting or electrospinning, and the prepared conductive thin film has a smooth surface and a resistance close to PBS.

In one embodiment, the cells to be treated are seeded on the conductive film, and receive the electric shock on the conductive film directly without the need to be pre-prepared as a cell suspension.

According to the present invention, the electric shock has a pulse width in a range of 10-150 ns. Preferably, the pulse width is in a range of 20-100 ns, 30-80 ns, 40-70 ns, or 50-60 ns, and specifically about 10 ns, 15 ns, 20 ns, 25 ns, 30 ns, 35 ns, 40 ns, 45 ns, 50 ns, 55 ns, 60 ns, 65 ns, 70 ns, 75 ns, 80 ns, 85 ns, 90 ns, 95 ns, 100 ns, 105 ns, 110 ns, 115 ns, 120 ns, 125 ns, 130 ns, 135 ns, 140 ns, 145 ns, or 150 ns.

In yet another embodiment, the field strength is in the range of 1-30 kV/cm. Preferably, the field strength is about 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm, or 30 kV/cm.

It should be noted that, the values of pulse width and field strength above not only refer to the values themselves, in some embodiments, the applied pulse width may have a certain variation from these values, which may be 1%, 2%, 3%, 4%, 5%, 6%, 7%, 8%, 9% or 10%, resulting from the precision of the device to apply the electric pulse and other reasons, therefore, in these embodiments, the values of pulse width and field strength will also include the variation around these values. Besides, in the present disclosure, such variation may be expressed by using "about".

In the term "nanosecond pulsed electric field", one nanosecond is equal to one billionth of a second, and a pulse which lasts from a several to tens of nanoseconds is called a nanosecond pulse. Nanosecond pulsed electric fields are mainly characterized by high power but low heat, so there is almost no heat generated during the treatment of tumors. In addition, it can penetrate a cell membrane to act on the organelles in the cell, thereby affecting the biological function of the cell.

In contrast to the electroporation of cell membrane caused by a longer pulse, a nanosecond pulse generates extremely high power (several billions of watts), extremely short duration (nanoseconds) and high field strength (kV/cm), thus it can penetrate into a cell and act on the subcellular structure before the cell membrane is fully charged. Because the duration of a nanosecond pulse is shorter than the time to charge cell membrane, thus it almost does not affect the cell membrane, and will not cause electroporation of cell membrane.

### EXAMPLES

Those examples which are not encompassed by the claims are given for illustrative purposes.

### Materials and methods

Apparatus

| Name of the Instruments | Type NO. | Manufacturer |
|---|---|---|
| High Speed Refrigerated Centrifuge | 5702R | Eppendorf |
| Inverted Phase Contrast Microscope | CKX41-RC | Olympus |
| Microscope Component for Fluorescence | U-RFLT50 | Olympus |
| Clean Bench | DL-CJ-2ND | HDL |
| Electronic Balance | BS124S | Sartorius |
| Laser Confocal Microscope | LSM510 | Zeiss |
| High-Pressure Steam Sterilization Pot | MLS-3750 | SANYO |
| Enzyme Reader | 680 | Bio-rad |
| Water Purifier | Millipore | MERCK |
| Complete set for Western Blotting | Bio-rad | Bio-rad |
| Real-time PCR Instrument | Pikoreal 96 | Thermo |
| Ordinary PCR Instrument | MyCycler | Bio-rad |
| Spectrophotometer | ND1000 | Thermo |
| Chemiluminescence Imaging System | 5200 | Tanon |
| Ice-maker | FM50 | Snow |
| Vortex Mixer | Vortex-5 | Vortex |
| Enzyme Reader | Synergy4 | Biotek |
| Electroporation Cuvette | BTX | BTX |

### Isolation and Culture of Bone Marrow Mesenchymal Stem Cellscc

Reagents: DMEM (Gibco); DMEM Dulbecco's Modified Eagle Medium (Gibco); Trypsin (Invitrogen); fetal bovine serum (FBS, Gibco); Dexamethasone (Life Technologies); TGF-β3 (Peprotech); sodium pyruvate (Life Technologies); vitamin C (Vc, Sigma); ITS (Insulin Transferrin Selenium premix, Life Technologies); proline (SINOPHARM); sodium glycerophosphate (Sigma); fast green (Amresco); Alkaline Phosphatase (ALP) Assay Kit (NanJing JianCheng Bioengineering Institute); TritonX-100 (Sigma); BD Matrigel^{™} matrix (BD Biosciences Company, 354277); and mTeSR1 Complete Kit (Stemcell Company, 05850).

The culture medium for bone marrow mesenchymal stem cells was prepared by: adding 160 mL of DMEM stock solution and 40 mL of melted FBS, and then adding 200 µL of 1000×PS, to a sterilized culture flask using a sample injector. This whole process was operated inside a sterilized Clean Bench in a sterile environment.

### Experimental Steps

### (1) Isolation of porcine bone marrow mesenchymal stem cells

In the experiments, porcine bone marrow mesenchymal stem cells were used. The pigs used in the experiments were purchased from Animal Experiment Department of Beijing Fuwai Hospital. The procedures for use of animals were approved by the Animal Ethical Review in the Beijing Fuwai Hospital, and the animal experiments for all the studies were conducted according to the related regulations of the Ethics Committee of Peking University.

A pork leg was soaked in 75% alcohol for 20 minutes, and then transferred to the clean hood, and the medullary cavity was open by an electric drill. The bone marrow was washed out with PBS to a petri dish, until the washing solution became clear. The mixture in the petri dish was collected and transferred to a 15 mL centrifuge tube, subjected to centrifugation at 1,000 rpm for 10 min in a centrifuge, and then the cells were collected.

### (2) Cell culture

The precipitated cells were suspended in the culture medium of the bone marrow mesenchymal stem cells and cultured. The culture medium was changed half at the first day, and then changed completely after a rinse with PBS at three-day intervals. During the culture, the cells were observed, photographed by a microscope and recorded.

### (3) Subculture

When the cells reached 80% confluence, 2 mL of trypsin was added to the cell culture dish, and the dish was put in an incubator to digest for 3 minutes. Once suspension of the bone marrow mesenchymal cells was observed under the microscope, a culture medium of 2-fold volume of trypsin was added into the petri dish, the mixture was transferred into a 15 mL centrifuge tube, then centrifuged at a speed of 1,000 rpm for 5 minutes in a refrigerated centrifuge. The cells were collected, resuspended in the culture medium and counted by using a hemocytometer. The cells were cultured at a density of 5,000 cells/cm² with the culture medium changed twice every week.

### (4) Cell cryopreservation

The subcultured bone marrow mesenchymal cells were digested with trypsin and centrifuged, and the supernatant was discarded. Cell cryopreservation medium was added to the cells and then transferred into a cryogenic vial. The cryogenic vial was successively placed in a programmed cooling box at -20°C for 3 hours, a low-temperature freezer at -80°C overnight, and then a liquid nitrogen tank for long-term preservation.

To resuscitate the cells, the cryogenic vial was immersed into a water bath at 37°C with quickly shake to let the ice melt. In the subsequent experiments, the fifth-generation primary bone marrow mesenchymal stem cells were used.
Culture of Human Embryonic Stem Cell Line H9 (all references to Human ESC Line H9 in the following examples are only for illustrative purposes)

### (1) Plating

BD Matrigel^{™} matrix (BD Biosciences Company, 354277) was used for plating cells in a 6-well plate. Matrigel was diluted with pre-cooled DMEM/F-12 according to the instruction and added into a 6-well plate, 1 mL in each well. The plate was shaken slightly to let the Matrigel uniformly covered on the surface of the well, and then incubated at room temperature for 1 h.

### (2) Culture

Cells of human embryonic stem cell line H9 were cultured in a culture medium (mTeSR1 Complete Kit, Stemcell, 05850), with the medium changed every day. The growth of cells was assessed and monitored until the next subculture.

### (3) Subculture

The cells were washed with DMEM/F-12, then digested using a dispase at 37°C for about 4-7 minutes. After removing the dispase by aspiration, the dish was gently washed with DMEM/F-12. The cell colonies were removed using a serological glass pipette or a cell scraper, collected, centrifuged, resuspended and uniformly mixed, and then subcultured at a ratio of 1:6 to 1:10.

### Application of Electrical Stimulation Via Electroporation Cuvette

### (1) Electric pulse device

The electric pulse device can be connected and combined in any manner well-known to these skilled in the art. The nanosecond pulsed electric field device used in the examples of the present disclosure comprised a high voltage DC power supply, a coaxial transmission cable, a high-voltage probe and a cell-stimulating electroporation cuvette (any commercially available type of electroporation cuvettes may be used, for example, BTX^{™} cuvettes). The high voltage DC power supply provided a stimulation electric field, the coaxial transmission cable could compress electrical energy, the high-voltage probe allowed to form a nanosecond pulsed electric field, and the nanosecond level electric field could be applied to the electroporation cuvette. The electric fields constructed by the device of the present disclosure were pulsed electric fields with a pulse width of 100 nanoseconds. The output voltage from the high voltage DC power supply can be read on an oscilloscope. Changing the output voltage can adjust the strength of the nanosecond pulse field inside the electroporation cuvette to different values.

### (2) Electrical stimulation as well as induction and/or analysis

The cells were counted by a hemocytometer (porcine bone marrow mesenchymal stem cells or human embryonic stem cells). The cells were suspended in PBS to prepare a cell suspension at 100,000-1,500,000 cells/ml. 500-800 µL of the cell suspension was added into the electroporation cuvette. For the test groups, cells were simulated by a nanosecond pulsed electric field with specific parameters. After the completion of the electrical stimulation, the cells were collected by a low-speed centrifugation. Then the porcine bone marrow mesenchymal stem cells were cultured for about two weeks to differentiate into chondroblasts (pellet culture), osteoblasts and lipoblasts by chondrogenic induction, osteogenic induction and adipogenic induction (more details will be described below), respectively. In addition, within 4 h after the cells be electrically stimulated, a part of the electrically stimulated cells was taken out for necessary analysis and detection, such as the stemness gene detection (in the human embryonic stem cells and the porcine bone marrow mesenchymal stem cells) and the global DNA methylation level detection (in the porcine bone marrow mesenchymal stem cells), as shown in the examples.

### Application of Electrical Stimulation Via Conductive Film

The conductive films used in the examples were prepared by the blend-casting technique, using MWCNTs (multi-walled carbon nanotubes) and poly-L-lactic acid with a molecular weight of 100,000 and an intrinsic viscosity of 2.7 dL/g, as raw materials. Specifically, it included the steps: 1) dissolving 1 g of polylactic acid in 5 mL of chloroform until fully dissolved in 8 h; 2) dispersing 30 mg of carboxylated carbon nanotubes in 5 mL of chloroform; 3) performing water bath untrasound for 30 min on the carboxylated carbon nanotubes mixture of step 2), and adding into the poly-L-lactic acid solution of step 1), to allow the final concentration of poly-L-lactic acid was 10% by w/v; 4) after ultra-sonication for 20 min, casting the resultant onto a glass plate and standing overnight; 5) peeling off the film; and 6) placing the film into a vacuum oven at 35°C for one day to fully evaporate the solvent, and obtain a conductive film, having a smooth surface and a resistance similar to that of PBS.

The nanosecond pulsed electric field device using the conductive film included the following components: a high voltage DC power supply, a coaxial transmission cable, a high-voltage probe, a conductor piece and a cell-simulating conductive film. The high voltage DC power supply provided a simulation electric field, the coaxial transmission cable could compress electrical energy, the high-voltage probe allowed to form a nanosecond pulsed electric field, and the nanosecond level electric field was applied on the conductive film though the conductor pieces at the two side of the conductive film. In this test, the device was used to construct a pulsed electric field having a pulse width of 100 nanoseconds. The output voltage from the high voltage DC power supply could be read on an oscilloscope. Changing the output voltage can adjust the strength of the nanosecond pulse field on the conductive film.

Using the conductive film to apply electric shocks specifically included the following steps. Porcine bone marrow mesenchymal stem cells were cultured until cell confluence reached 80%. 2 mL of trypsin was added into the cell culture dish, and the cells were digested for 3 minutes in an incubator. Once suspension of the bone marrow mesenchymal cells was observed under the microscope, a cell culture medium containing 2-fold volume of the trypsin was added to the dish. The cells were collected and transferred into a 15 mL centrifuge tube for centrifugation at a speed of 1,000 rpm for 5 minutes in a refrigerated centrifuge. The collected cells were counted by a hemocytometer, and resuspended in a culture medium to a cell density of 500,000-4,000,000 cells/mL. 50 µL of the resulting suspension was dropped uniformly onto the conductive thin film, incubated at room temperature for 3-12 h, and then additional culture medium was added. The culture medium was changed twice a week. When applying electrical stimulation, the culture medium was changed to phosphate buffered saline (PBS) and the conductor pieces were connected to the two sides of the conductive film to apply nanosecond pulsed electric field stimulation having specific parameters. After the completion of the electrical stimulation, PBS was replaced back to growth medium for continuing culture. The cells were digested at a predetermined time point for the subsequent detection.

### Induction of Chondrogenic Differentiation

The culture medium for chondrogenic induction was formulated by: high glucose DMEM (Gibco), 10 ng/mL TGF-β3 (R&D Systems, Minneapolis, MN), 10⁻⁷ M dexamethasone (D4902, Sigma), 50 µg/mL ascorbic acid (A5960, Sigma), 1 mM sodium pyruvate (P2256, Sigma), 4 mM proline (P5607, Sigma) and 1% (v/v) ITS (41400045, Gibco).

The culture was performed by the steps: digesting bone marrow mesenchymal stem cells by trypsin and counting; removing the culture medium, changing to the culture medium for chondrogenic induction to prepare a cell suspension at 2.5×10⁵ cells/mL; transferring 1 mL of the suspension to a 15 mL centrifuge tube to centrifuge at a speed of 500× g for 5 min; culturing the stem cells by pellet culture for 14 days with the culture medium changed every 3 days.

### Induction of Adipogenic Differentiation

The culture medium for adipogenic induction was formulated by the solution A and solution B.

The solution A was a differentiation medium, prepared by 175 mL of a differentiation medium for adipogenic induction of bone marrow mesenchymal stem cells (as a base culture medium of solution A), 20 mL of fetal bovine serum, 2 mL of Penicillin-Streptomycin, 2 mL of Glutamine, 400 µL of Insulin, 200 µL of IBMX, 200 µL of Rosiglitazone, and 200 µL of Dexamethasone.

The solution B was a maintenance medium, prepared by 175 mL of a differentiation medium for adipogenic induction of bone marrow mesenchymal stem cells (as a base culture medium of solution B), 20 mL of fetal bovine serum, 2 mL of Penicillin-Streptomycin, 2 mL of Glutamine, and 400 µL of Insulin.

The culture was performed by the following steps.

Bone marrow mesenchymal stem cells were cultured in an incubator at 37°C with 5% of CO₂. When the cell confluence reached 80-90%, the cells were digested by using 0.25% trypsin-0.04% EDTA. The digested mesenchymal stem cells were seeded at a cell density of 2×10⁴ cells/cm² in a 6-well plate, with 2 mL of complete medium at each well. The cells were cultured in an incubator at 37°C with 5% of CO₂, with the medium changed at a three-day interval, until the cell confluence reached 100% or over confluence.

The complete medium for the mesenchymal stem cells was removed carefully by aspiration, and 2 mL of the solution A (differentiation medium for adipogenic induction of bone marrow mesenchymal stem cells) was added into the 6-well plate. After induction for 3 days, the solution A was removed from the 6-well plate by aspiration, and 2 mL of the solution B (differentiation medium for adipogenic induction of bone marrow mesenchymal stem cells) was added. After 24 h, the solution B was removed by aspiration and replaced with solution A for induction.

After alternately culturing the cells with the solution A and the solution B for 3-5 rounds (12-20 days), the solution B was used again to maintain the culture for 4-7 days until the lipid droplets became large and round enough. When the cells were maintained in solution B, it was necessary to change to fresh solution B at a 2-3 days' interval.

### Induction of Osteogenic Differentiation

The osteogenic induction medium was formulated by DMEM culture medium, containing 10% of FBS, 100 nM dexamethasone, 10 mM β-sodium glycerophosphate, and 50 µg/mL vitamin C.

The culture was performed by the following steps: digesting bone marrow mesenchymal stem cells by trypsin and counting; diluting the cell suspension to a concentration of 50,000 cells/mL, adding 100 µL of the cell suspension into a 96-well plate; when the cell confluence reached about 70%, removing the culture medium and washing the cells with PBS for 3 times; and adding the osteogenic induction medium to induce the osteogenic differentiation of the bone marrow mesenchymal stem cells, with the culture medium changed every 3 days.

### Detection of Chondrogenic Differentiation, Adipogenic Differentiation and Osteogenic Differentiation

### (1) Detection of chondrogenic differentiation

A. A genetic detection was performed to detect gene expression of COL I, COL II, COL X, SOX 9, ACAN. COL II, SOX 9 and ACAN are the functional genes favorable to chondrogenesis, while COL I and COL X are hypertrophic genes not favorable to chondrogenesis.
B. In the histology detection, the pellets were fixed in 4% paraformaldehyde, embedded in agarose, dehydrated and transparentized, embedded in paraffin, and sliced. Then, alcian blue dye was used to stain the extracellular matrix, and HE-staining was used to show the cell morphology.
C. Semi-quantitative detection based on glycosaminoglycan (GAG) was performed by: digesting the cell pellets by using proteinase K at 56°C overnight; heating the sample at 90°C for 10 min; using cationic dye, DMMB, to complex with GAG for half-hour; using a decomplexation solution to dissociate the precipitation; detecting absorbance at a wavelength of 656 nm; and calculating the GAG content in the extracellular matrix.

### (2) Detection of osteogenic differentiation

Alizarin red method was performed as follows: adjusting pH of alizarin red solution to 4.0-4.2; staining the sample for 1 h at room temperature; incubating in PBS for 15 min and removing the supernatant; washing the sample with PBS for 3 times and thoroughly removing the supernatant; adding hexadecyl chloride; and standing at room temperature for 30 min. The supernatant was pipetted out and analyzed by using the ultraviolet spectrophotometer. The absorbance of the sample at a wavelength of 562 nm was measured, and a hexadecyl chloride solution was used as blank control to set zero value. Additionally, the absorbance of the supernatant from a group of the cells without addition of alizarin red were measured. The measurement for each sample was repeated for 3 times to calculate average value and standard deviation.

### (3) Detection of adipogenic differentiation

This detection was performed by: staining the sample with oil red O for 1 h; washing with PBS for twice; adding isopropyl alcohol for isolation; and measuring at 490 nm for semi-quantitative detection.

### Detection of Stemness-Related Genes and Methylation-Related Genes

Reagents: TRNzol Total RNA Isolation Kit (DP405), FastQuant RT Kit (with gDNase) (KR106) and SuperReal PreMix Plus (SYBR Green) (FP205) were purchased from Tiangen Biotech (Beijing) co., Ltd. Isopropyl alcohol and chloroform were purchased from Beijing Reagent Factory.

### Procedure

(1) RNA isolation and reverse transcription were performed by: extracting RNA by using DP405 product from Tiangen Company, and subsequently performing reverse transcription by using KR106 kit from Tiangen Company according to the instructions.
(2) Real-time PCR detection was performed by: carrying out quantitative real-time PCR by using FP205 kit from Tiangen Company to detect the level of gene expression of Oct4, CARM1, DNMT1, DNMT3A, DNMT3B, Mll1, Mll2, Nanog, PRMT5, Sox2 and so on.

### Detection of DNA Methylation Level

Reagents: MethylFlash Global DNA Methylation (5-mC) ELISA Easy Kit (p-1030) (Epigentek Company) was used.

### Detection method

Standard curve was plotted according to the instruction of the kit, and DNA methylation levels of samples were detected.

### Example 1 Electrical Stimulation

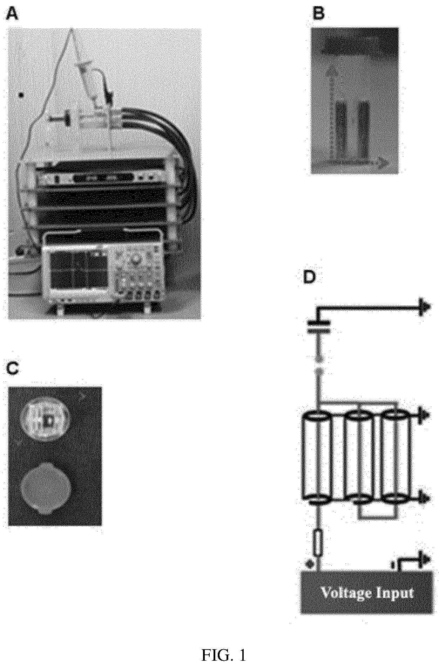

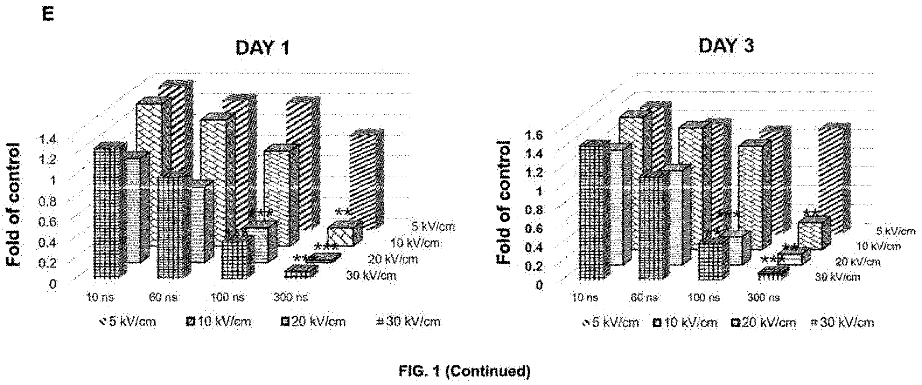

When using nanosecond pulsed electric fields to stimulate cells, the primary parameters are pulse width and field strength, which need to be controlled at a suitable range to achieve the aim of the experiment and to ensure cell viability (J.C. Weaver, etc.,"A brief overview of electroporation pulse strength-duration space: A region where additional intracellular effects are expected", Bioelectrochemistry, 87 (2012) 236-243; Tadej Kotnik, etc., Electroporation-based applications in biotechnology, Trends in Biotechnology, 33(8), 2015.8). In this Example, cells were subjected to electric shock via the electroporation cuvette (FIG. 1 A-D), with the pulse width being adjusted within the range of 1-300 ns, and the field strength being adjusted within the range of 1-30 kV/cm. The results showed that, within the above ranges, an electric shock with the combination of the pulse width and the field strength did not have obviously adverse effect on the cell survival (FIG. 1E). This is consistent with the current understanding for the effect of pulse width and field strength of nanosecond pulsed electric fields on cell survival in this field (J.C. Weaver, etc., "A brief overview of electroporation pulse strength-duration space: A region where additional intracellular effects are expected", Bioelectrochemistry, 87 (2012) 236-243; Tadej Kotnik, etc., Electroporation-based applications in biotechnology, Trends in Biotechnology, 33(8), 2015.8).

In the subsequent experiments, cells were electrically stimulated by the fields with the combination of the two parameters. In the experiments, as needed, the stimulation was carried out at times ranging from 1 to 1,000 times, at a frequency ranging from 1 to 1,000 Hz, at room temperature or particularly at a suitable temperature ranging from 20 to 37°C. Multiple combinations can produce certain effects.

As for porcine bone marrow mesenchymal stem cells, the parameters enabling a better effect included three groups (group A, B and C) as shown below. For convenience, in the subsequent examples, the group A, group B and group C might be used to represent parameters described below.
Group A: pulse width 10 ns, field strength 20 kV/cm;
Group B: pulse width 100 ns, field strength 10 kV/cm;
Group C: pulse width 60 ns, field strength 5 kV/cm; and
stimulation was performed for 5 times, at a frequency of 1 Hz, at room temperature.

As for human embryonic stem cells, the stimulation experiments were also conducted with the pulse width within the range of 1-300 ns and the field strength within the range of 1-30 kV/cm. The cells were stimulated under different combinations of parameters. In Example 4, six combinations: 10 ns+5 kV/cm, 10 ns+10 kV/cm, 10 ns+20 kV/cm, 100 ns+5 kV/cm, 100 ns+10 kV/cm and 100 ns+20 kV/cm, were specifically tested. After the stimulations under these six conditions, the expression of the target genes was detected.

It should be noted that the specific parameter combinations above merely are part of the preferred embodiments. In facts, within the conditions described above for applying nanosecond pulsed electric fields, there may be a variety of suitable parameter combinations. Furthermore, for the different types of the stem cells, the most suitable conditions may be also different, and need to determine specific parameter combinations depending on the different target cells. However, due to these skilled in the art have already know clearly about the range of the conditions to apply the nanosecond pulse, which is further supported by the present disclosure, it can be understood that these skilled in the art are possible to acquire desired parameter combinations, based on the content of the present disclosure.

### Example 2 Nanosecond Pulsed Electric Field Stimulation Increases Stemness of Mesenchymal Stem Cells

Nanosecond pulsed electric fields increases the expression of stemness-related genes.

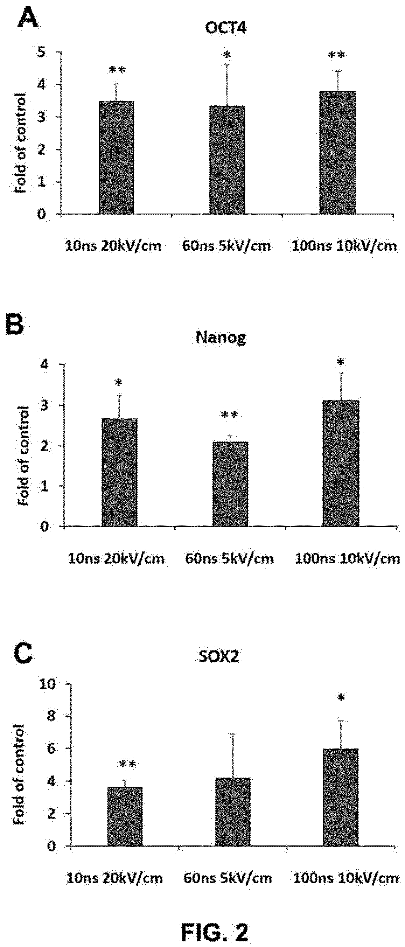

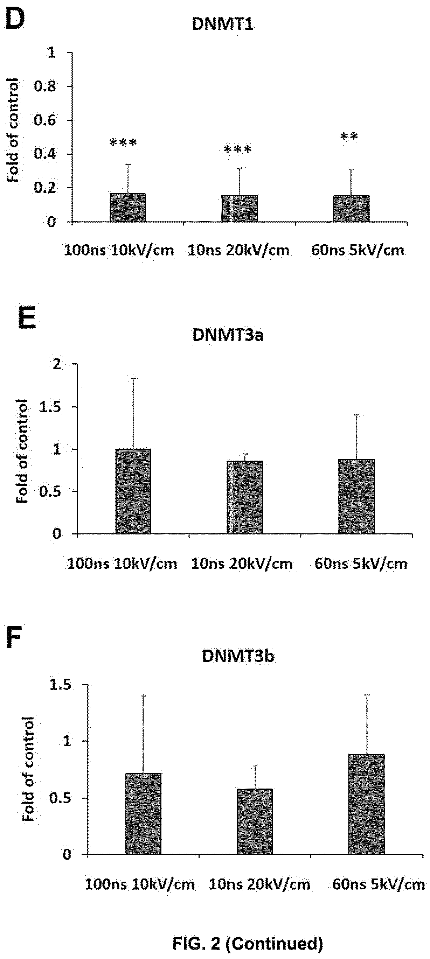

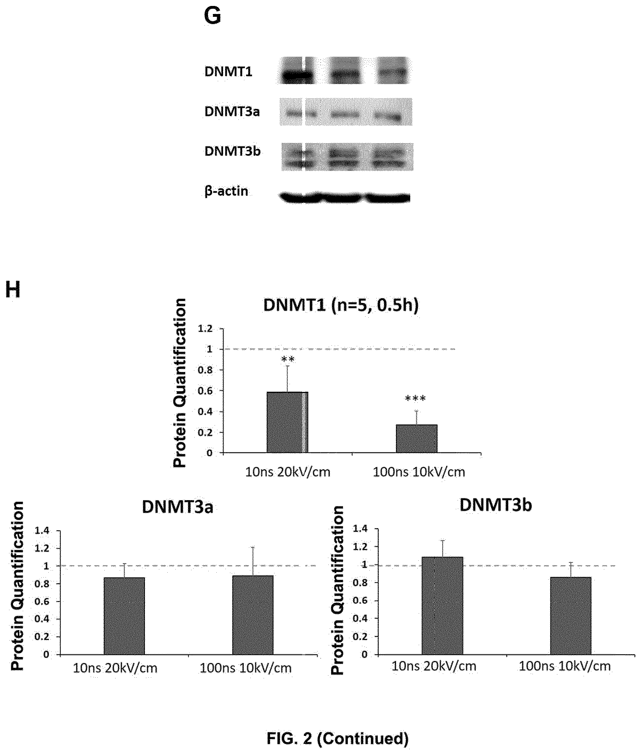

It is known that electrical signal stimulation may facilitate stem cell differentiation (e.g., mesenchymal stem cell). The reason is generally speculated to be that electrical signals can activate signals or pathways related to cell differentiation, thereby promoting the ability of stem cells to differentiate into a variety of tissue cells. However, because the mechanism of stem cell differentiation is very complicated, the reasons for how the external stimulations enhance the differentiate capability of the stem cells are still poorly understood. The inventors unexpectedly found that when detecting the expression of genes related to stemness of cells after nanosecond pulsed electric field stimulation (the experiment was repeated for 5 times, and the cells were collected 0.5 h to 2 h after the electrical stimulation), the expression levels of the genes important for maintaining stemness were increased significantly. As shown in FIG. 2A-2C, as compared with the control group without the electric field treatment, the expression level of OCT4 gene increased by nearly 3 times, and the expression level of NANOG and SOX2 also increased significantly. These results indicated that after the mesenchymal stem cells were simulated by nanosecond pulsed electric fields, the stemness of the cells was increased.

It is known that DNA methylation plays an important role on stemness maintenance and differentiation of cells. For example, a cell with higher stemness generally has higher DNA methylation level; in contract, a decrease in DNA methylation is accompanied with differentiation of cell. The expression of methylation genes is closely related with the methylation level of cells. In order to further investigate the increase of the stemness of mesenchymal stem cells after nanosecond pulsed electric field stimulation, the expression of the genes related to methylation in cells was detected. The results show that the expression levels of DNMT1 (closely related to methylation level in cells) mRNA and protein were decreased significantly (FIG. 2D-2H), but DNMT3a and DNMT3b are not affected, indicating that the effect of the electric shock on methylation is achieved by specifically affecting the expression of DNMT1.

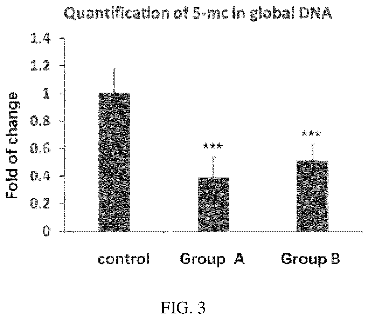

Subsequently, in order to analyze the DNA methylation state in cells directly, MethylFlash Global DNA Methylation (5-mC) ELISA Easy Kit was used to detect the global DNA methylation level in cells after stimulation. The results show that nanosecond pulsed electric fields stimulation reduced the global DNA methylation level by 40-80% (FIG. 3).

The results above demonstrated that nanosecond pulsed electric field stimulation could increase stemness of stem cells. The present disclosure is the first to show that nanosecond pulsed electric fields can increase the cell stemness.

### Example 3 Electric Shock Significantly Enhances the Ability of Mesenchymal Stem Cells for Chondrogenic Differentiation, Osteogenic Differentiation and Adipogenic Differentiation

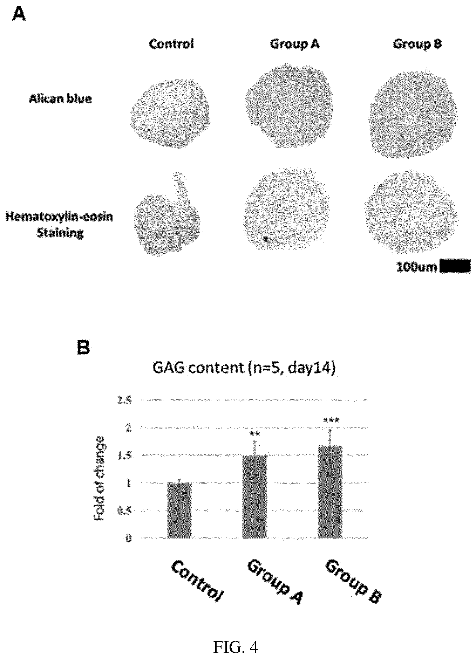

In this Example, the changes on the differentiation capability of the mesenchymal stem cells after nanosecond pulsed electric field simulation were detected. For the detection of chondrogenic differentiation, the alcian blue staining was conducted on the 14th day after stimulation. The results showed that after the cells were pretreated with nanosecond pulsed electric fields and then subjected to chondrogenic induction culture, the extracellular matrix secretion is increased obviously (FIG. 4A).

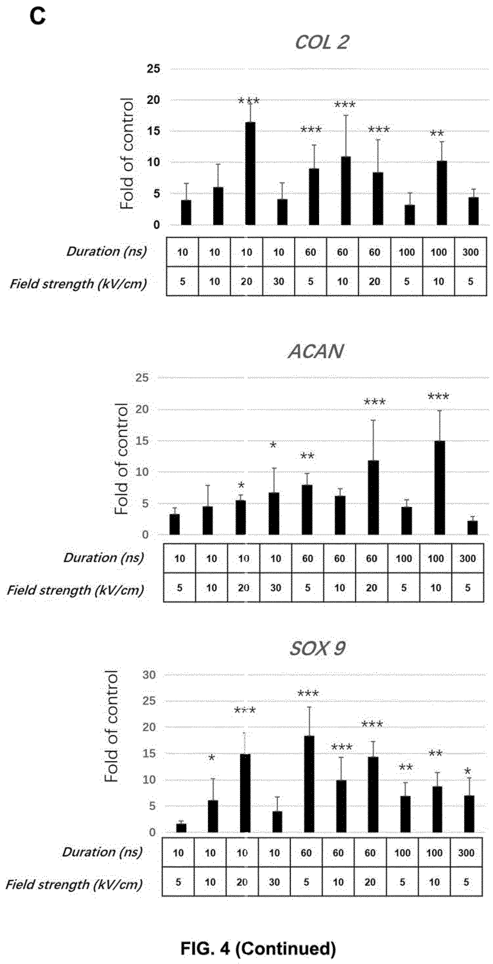

Dimethylmethylene Blue (DMMB) assay was used to quantify glycosaminoglycan, and it was found that GAG content was increased to about 1.5 folds (FIG. 4B). Further, the expressions of COL II, ACAC and SOX 9 genes, which can significantly facilitate the function of cartilage, were detected. It was found that the expressions of the three genes were increased significantly (FIG. 4C), while the expressions of COL I, COL X were not affected significantly (results not shown). These results demonstrate that the capability of mesenchymal stems for chondrogenic differentiation is enhanced significantly.

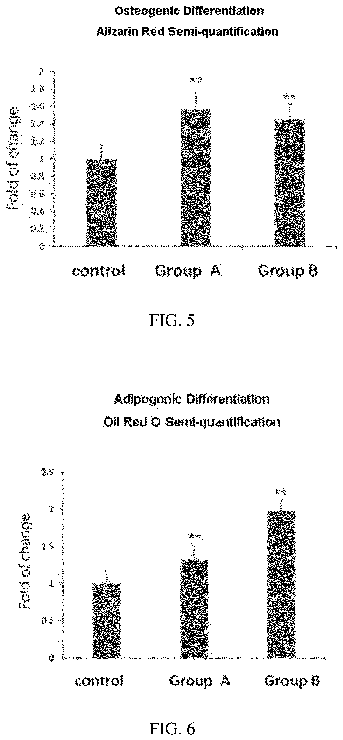

The changes on ability for osteogenic differentiation, after the mesenchymal stem cells was simulated by nanosecond pulsed electric fields, was studied. After the cells were pretreated with nanosecond pulsed electric fields and then subjected to osteogenic induction culture, the efficiency of osteogenic induction was increased to about 1.5 fold, compared with the use of induction medium only. This experiment was repeated for 3 times (n=3) (FIG. 5).

After the cells were pretreated with nanosecond pulsed electric fields and then subjected to adipogenic induction culture, compared with the use of an induction medium only, the efficiency of adipogenic induction was increased significantly. This experiment was repeated for 3 times (n=3) (FIG. 6).

The results above show that the increase of stemness of the mesenchymal stem cells after nanosecond pulsed electric field stimulation leads to direct enhancement on differentiation capability. This also indicates that using nanosecond pulsed electric field to stimulate the stem cells has a broad practical application.

### Example 4 Nanosecond Pulsed Electric Fields Enhances Stemness of Embryonic Stem Cells

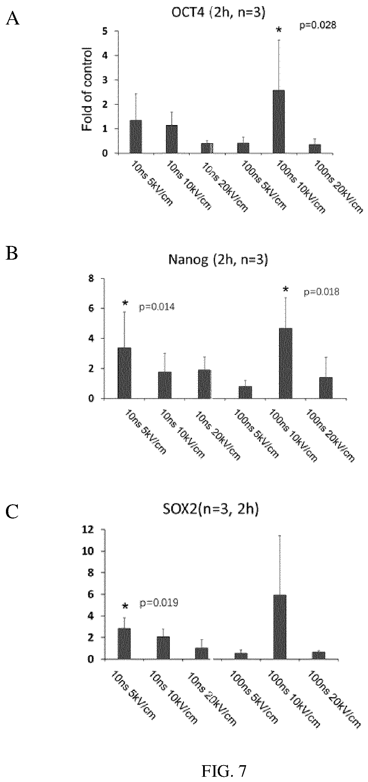

In order to investigate the effect of nanosecond pulsed electric fields on the stemness of other stem cell types, the human embryonic stem cells were treated with nanosecond pulses under different combinations of pulse width and field strength. Specifically, six combinations were tested, that was, 10 ns+5 kV/cm, 10 ns+10 kV/cm, 10 ns+20 kV/cm, 100 ns+5 kV/cm, 100 ns+10 kV/cm and 100 ns+20 kV/cm. The results show that with a pulse width of 100 ns and a field strength of 10 kV/cm, the nanosecond pulsed electric fields are able to increase the expression levels of OCT4, NANOG significantly; and with a pulse width of 10 ns and a field strength of 5 kV/cm, the nanosecond pulsed electric fields also are able to increase the expression level of NANOG and SOX2 genes (FIG. 7A-7C) significantly. These results demonstrate that after the nanosecond pulsed electric field simulation, the stemness of embryonic stem cells is enhanced effectively.

### Example 5 Applying Electrical Stimulation Via Conductive Film More Effectively Enhances the Stemness of Stem Cells

Applying nanosecond pulsed electric fields using an electroporation cuvette has the following disadvantages: it only allow single action but not continuous actions at multiple time points; the electric field only acts on a limited amount of cells; and electroporation cuvette is only suitable for suspended single cells, not for cells in pellet or adherent cell, thus the cells need to be digested to single cell, which may affect the cell state; in addition, electroporation cuvette cannot be used for tissues *in vivo.*

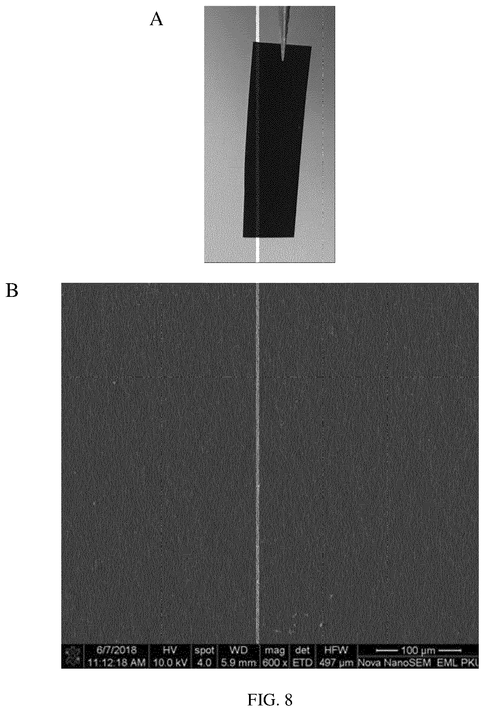

Thus, the inventors have improved the manner of applying electric fields, that is, a conductive film (FIG. 8) is used to replace the electroporation cuvette. In this example, the effect of using a conductive film to apply an electric field on porcine bone marrow mesenchymal stem cells was compared with that of using an electroporation cuvette.

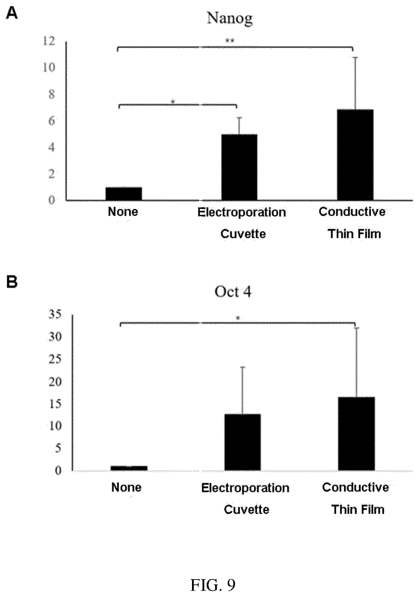

The electric pulse with a parameter of 100 ns and 10 kV/cm was used to simulate the cell suspension in an electroporation cuvette and the porcine bone marrow mesenchymal stem cells seeded on a conductive film, respectively, and the expressions of stemness genes Nanog and OCT 4 were detected. It was found that either applying an electric shock directly to the cells seeded on an conductive film or applying an electric shock to the cell suspension via an electroporation cuvette can enhance the expression of stemness genes effectively, whereas using a conductive film to apply an electric shock can achieve an even higher enhancement, and in particular, the gene expression of Nanog is enhanced more significantly (FIG. 9,** represents a very significant difference for p<0.01; and * represents a significant difference for p<0.05). In addition to the advantage on the electric shock effects, using an conductive film to apply an electric shock is further advantaged at that on one hand there is no need to digest cells to prepare suspension, which saves the cost and time for operations, and on other hand cell viability is preserved maximally, which enable the cells after the electric shock to be used in subsequent treatments, for example, the cells adhered on a conductive film after the electric shock can continue to be used in electric shock after a temporary culture, thereby the flexibility of operation is greatly improved. This also indicates that using a conductive film to apply nanosecond pulsed electric fields has good application prospect.

## Claims

1. Use of an electric field in enhancing stemness of a pluripotent stem cell, an embryonic stem cell or an iPS cell *in vitro,* increasing the expression of stemness-related genes in a pluripotent stem cell, an embryonic stem cell or an iPS cell *in vitro,* reducing the expression of methylation-related genes in a pluripotent stem cell, an embryonic stem cell or an iPS cell *in vitro* and/or reducing the methylation level in a pluripotent stem cell, an embryonic stem cell or an iPS cell *in vitro;*
wherein the electric field is a nanosecond pulsed electric field (nsPEF) having a pulse width in a range of 10-150 ns.

2. The use according to claim 1, wherein the electric field is applied to the cell to give an electric shock to the cell.

3. The use according to claim 1 or 2, wherein the nanosecond pulsed electric field has a field strength in a range of 1-30 kV/cm.

4. The use according to claim 3, wherein the pulse width is in a range of 20-100 ns, 30-80 ns, 40-70 ns, or 50-60 ns.

5. The use according to claim 3 or 4, wherein the field strength is about 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm, or 30 kV/cm.

6. The use according to claim 1 or 2, wherein the electric shock is applied 1-1000 times, 10-900 times, 20-800 times, 30-700 times, 40-600 times, 50-500 times, 60-400 times, 70-300 times, 80-200 times, or 90-100 times; and/or
frequency of the electric shock is 1-1000 Hz, 10-900 Hz, 20-800 Hz, 30-700 Hz, 40-600 Hz, 50-500 Hz, 60-400 Hz, 70-300 Hz, 80-200 Hz, or 90-100 Hz.

7. A method for enhancing stemness of a pluripotent stem cell, an embryonic stem cell or an iPS cell *in vitro,* increasing the expression of stemness-related genes in a pluripotent stem cell, an embryonic stem cell or an iPS cell *in vitro,* reducing the expression of methylation genes and/or reducing the methylation level in a pluripotent stem cell, an embryonic stem cell, or an iPS cell *in vitro,* comprising applying an electric field to the cell to give an electric shock to the cell;
wherein the electric field is a nanosecond pulsed electric field having a pulse width in a range of 10-150 ns.

8. The method according to claim 7, wherein the nanosecond pulsed electric field has a field strength in a range of 1-30 kV/cm.

9. The method according to claim 8, wherein the pulse width is in a range of 20-100 ns, 30-80 ns, 40-70 ns, or 50-60 ns.

10. The method according to claim 8 or 9, wherein the field strength is about 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm, or 30 kV/cm.

11. The method according to claim 7, wherein the electric shock is applied 1-1000 times, 10-900 times, 20-800 times, 30-700 times, 40-600 times, 50-500 times, 60-400 times, 70-300 times, 80-200 times, or 90-100 times; and/or
frequency of the electric shock is 1-1000 Hz, 10-900 Hz, 20-800 Hz, 30-700 Hz, 40-600 Hz, 50-500 Hz, 60-400 Hz, 70-300 Hz, 80-200 Hz, or 90-100 Hz.

12. The method according to any one of claims 7 to 11, wherein the electric field is applied via an electroporation cuvette.

13. The method according to any one of claims 7 to 11, wherein the electric field is applied via a conductive film; and
preferably, the conductive film is prepared from poly-L-lactic acid (PLLA) and multi-walled carbon nanotubes (WCNTs) by blend-casting or electrospinning.

14. The method according to claim 13, wherein the conductive film is prepared by blend-casting, comprising:
1) dissolving the poly-L-lactic acid in a solvent until fully dissolved;
2) dispersing the multi-walled carbon nanotubes in the same solvent;
3) performing ultrasonication on the multi-walled carbon nanotubes mixture of step 2), and adding into the poly-L-lactic acid solution of step 1), to allow the final concentration of poly-L-lactic acid is 1-50% by w/v;
4) after ultrasonication, casting the resultant on a plate and standing;
5) peeling off the film formed on the plate; and
6) placing the film into an oven to fully evaporate the solvent, and obtain a conductive film.

15. The method according to claim 13, wherein the poly-L-lactic acid has a molecular weight in a range of 10,000-1,000,000 daltons, and an intrinsic viscosity of 1-20 dL/g.

## Patentansprüche

1. Verwendung eines elektrischen Feldes zum Erhöhen der Stammzellfähigkeit einer pluripotenten Stammzelle, einer embryonalen Stammzelle oder einer iPS-Zelle *in vitro,* Erhöhen der Expression von Genen, die mit Stammzellfähigkeit in Zusammenhang stehen, in einer pluripotenten Stammzelle, einer embryonalen Stammzelle oder einer iPS-Zelle *in vitro,* Verringern der Expression von Genen, die mit Methylierung in Zusammenhang stehen, in einer pluripotenten Stammzelle, einer embryonalen Stammzelle oder einer iPS-Zelle *in vitro* und/oder Verringern des Methylierungsniveaus in einer pluripotenten Stammzelle, einer embryonalen Stammzelle oder einer iPS-Zelle *in vitro;*
wobei das elektrische Feld ein im Nanosekundenbereich gepulstes elektrisches Feld (nsPEF) mit einer Pulsbreite in einem Bereich von 10-150 ns ist.

2. Verwendung gemäß Anspruch 1, wobei das elektrische Feld an die Zelle angelegt wird, um der Zelle einen Elektroschock zu versetzen.

3. Verwendung gemäß Anspruch 1 oder 2, wobei das im Nanosekundenbereich gepulste elektrische Feld eine Feldstärke in einem Bereich von 1-30 kV/cm aufweist.

4. Verwendung gemäß Anspruch 3, wobei die Pulsbreite in einem Bereich von 20-100 ns, 30-80 ns, 40-70 ns oder 50-60 ns liegt.

5. Verwendung gemäß Anspruch 3 oder 4, wobei die Feldstärke etwa 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm oder 30 kV/cm beträgt.

6. Verwendung gemäß Anspruch 1 oder 2, wobei der Elektroschock 1-1000-mal, 10-900-mal, 20-800-mal, 30-700-mal, 40-600-mal, 50-500-mal, 60-400-mal, 70-300-mal, 80-200-mal oder 90-100-mal appliziert wird; und/oder
die Frequenz des Elektroschocks 1-1000 Hz, 10-900 Hz, 20-800 Hz, 30-700 Hz, 40-600 Hz, 50-500 Hz, 60-400 Hz, 70-300 Hz, 80-200 Hz oder 90-100 Hz beträgt.

7. Verfahren zum Erhöhen der Stammzellfähigkeit einer pluripotenten Stammzelle, einer embryonalen Stammzelle oder einer iPS-Zelle *in vitro,* Erhöhen der Expression von Genen, die mit Stammzellfähigkeit in Zusammenhang stehen, in einer pluripotenten Stammzelle, einer embryonalen Stammzelle oder einer iPS-Zelle *in vitro,* Verringern der Expression von Methylierungsgenen und/oder Verringern des Methylierungsniveaus in einer pluripotenten Stammzelle, einer embryonalen Stammzelle oder einer iPS-Zelle *in vitro,* wobei das Verfahren ein Anlegen eines elektrischen Feldes an die Zelle, um der Zelle einen Elektroschock zu versetzen, umfasst;
wobei das elektrische Feld ein im Nanosekundenbereich gepulstes elektrisches Feld mit einer Pulsbreite in einem Bereich von 10-150 ns ist.

8. Verfahren gemäß Anspruch 7, wobei das im Nanosekundenbereich gepulste elektrische Feld eine Feldstärke in einem Bereich von 1-30 kV/cm aufweist.

9. Verfahren gemäß Anspruch 8, wobei die Pulsbreite in einem Bereich von 20-100 ns, 30-80 ns, 40-70 ns, oder 50-60 ns liegt.

10. Verfahren gemäß Anspruch 8 oder 9, wobei die Feldstärke etwa 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm oder 30 kV/cm beträgt.

11. Verfahren gemäß Anspruch 7, wobei der Elektroschock 1-1000-mal, 10-900-mal, 20-800-mal, 30-700-mal, 40-600-mal, 50-500-mal, 60-400-mal, 70-300-mal, 80-200-mal oder 90-100-mal appliziert wird; und/oder
die Frequenz des Elektroschocks 1-1000 Hz, 10-900 Hz, 20-800 Hz, 30-700 Hz, 40-600 Hz, 50-500 Hz, 60-400 Hz, 70-300 Hz, 80-200 Hz oder 90-100 Hz beträgt.

12. Verfahren gemäß einem jeglichen der Ansprüche 7 bis 11, wobei das elektrische Feld über eine Elektroporationsküvette angelegt wird.

13. Verfahren gemäß einem jeglichen der Ansprüche 7 bis 11, wobei das elektrische Feld über eine leitfähige Folie angelegt wird; und
vorzugsweise die leitfähige Folie aus Poly-L-Milchsäure (PLLA) und mehrwandigen Kohlenstoff-Nanoröhren (WCNTs) durch Mischgießen oder Elektrospinnen hergestellt wird.

14. Verfahren gemäß Anspruch 13, wobei die leitfähige Folie durch Mischgießen hergestellt wird, umfassend:
1) Lösen der Poly-L-Milchsäure in einem Lösungsmittel, bis sie vollständig gelöst ist;
2) Dispergieren der mehrwandigen Kohlenstoff-Nanoröhren in dem gleichen Lösungsmittel;
3) Durchführen einer Ultraschallbehandlung auf das Gemisch von mehrwandigen Kohlenstoff-Nanoröhrchen von Schritt 2) und Hinzufügen zu der Poly-L-Milchsäure-Lösung von Schritt 1), so dass ermöglicht wird, dass die Endkonzentration der Poly-L-Milchsäure 1-50% nach Gew./Vol. beträgt;
4) nach der Ultraschallbehandlung Gießen des Ergebnisses auf eine Platte und Stehenlassen;
5) Abziehen der auf der Platte gebildeten Folie; und
6) Platzieren der Folie in einen Ofen, um das Lösungsmittel vollständig zu verdampfen und eine leitfähige Folie zu erhalten.

15. Verfahren gemäß Anspruch 13, wobei die Poly-L-Milchsäure ein Molekulargewicht in einem Bereich von 10 000-1 000 000 Dalton und eine intrinsische Viskosität von 1-20 dL/g aufweist.

## Revendications

1. Utilisation d'un champ électrique dans l'amélioration du caractère souche d'une cellule souche pluripotente, d'une cellule souche embryonnaire ou d'une cellule iPS *in vitro,* l'augmentation de l'expression de gènes liés au caractère souche dans une cellule souche pluripotente, une cellule souche embryonnaire ou une cellule iPS *in vitro,* la réduction de l'expression de gènes liés à la méthylation dans une cellule souche pluripotente, une cellule souche embryonnaire ou une cellule iPS *in vitro* et/ou la réduction du niveau de méthylation dans une cellule souche pluripotente, une cellule souche embryonnaire ou une cellule iPS *in vitro ;*
dans laquelle le champ électrique est un champ électrique pulsé en nanoseconde (nsPEF) comportant une largeur d'impulsion comprise dans une plage de 10 à 150 ns.

2. Utilisation selon la revendication 1, dans laquelle le champ électrique est appliqué à la cellule pour donner un choc électrique à la cellule.

3. Utilisation selon l'une des revendications 1 ou 2, dans laquelle le champ électrique pulsé en nanoseconde comporte une intensité de champ comprise dans une plage de 1 à 30 kV/cm.

4. Utilisation selon la revendication 3, dans laquelle la largeur d'impulsion est comprise dans une plage de 20 à 100 ns, 30 à 80 ns, 40 à 70 ns ou 50 à 60 ns.

5. Utilisation selon l'une des revendications 3 ou 4, dans laquelle l'intensité de champ est d'environ 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm ou 30 kV/cm.

6. Utilisation selon l'une des revendications 1 ou 2, dans laquelle le choc électrique est appliqué 1 à 1000 fois, 10 à 900 fois, 20 à 800 fois, 30 à 700 fois, 40 à 600 fois, 50 à 500 fois, 60 à 400 fois, 70 à 300 fois, 80 à 200 fois ou 90 à 100 fois ; et/ou
la fréquence du choc électrique est de 1 à 1000 Hz, 10 à 900 Hz, 20 à 800 Hz, 30 à 700 Hz, 40 à 600 Hz, 50 à 500 Hz, 60 à 400 Hz, 70 à 300 Hz, 80 à 200 Hz ou 90 à 100 Hz.

7. Procédé permettant l'amélioration du caractère souche d'une cellule souche pluripotente, d'une cellule souche embryonnaire ou d'une cellule iPS *in vitro,* l'augmentation de l'expression de gènes liés au caractère souche dans une cellule souche pluripotente, une cellule souche embryonnaire ou une cellule iPS *in vitro,* la réduction de l'expression de gènes de méthylation et/ou la réduction du niveau de méthylation dans une cellule souche pluripotente, une cellule souche embryonnaire ou une cellule iPS *in vitro,* comprenant l'application d'un champ électrique à la cellule pour administrer un choc électrique à la cellule ;
dans lequel le champ électrique est un champ électrique pulsé en nanoseconde comportant une largeur d'impulsion comprise dans une plage de 10 à 150 ns.

8. Procédé selon la revendication 7, dans lequel le champ électrique pulsé en nanoseconde présente une intensité de champ comprise dans une plage de 1 à 30 kV/cm.

9. Procédé selon la revendication 8, dans lequel la largeur d'impulsion est comprise dans une plage de 20 à 100 ns, 30 à 80 ns, 40 à 70 ns, ou 50 à 60 ns.

10. Procédé selon l'une des revendications 8 ou 9, dans lequel l'intensité de champ est d'environ 1 kV/cm, 2 kV/cm, 3 kV/cm, 4 kV/cm, 5 kV/cm, 6 kV/cm, 7 kV/cm, 8 kV/cm, 9 kV/cm, 10 kV/cm, 11 kV/cm, 12 kV/cm, 13 kV/cm, 14 kV/cm, 15 kV/cm, 16 kV/cm, 17 kV/cm, 18 kV/cm, 19 kV/cm, 20 kV/cm, 21 kV/cm, 22 kV/cm, 23 kV/cm, 24 kV/cm, 25 kV/cm, 26 kV/cm, 27 kV/cm, 28 kV/cm, 29 kV/cm ou 30 kV/cm.

11. Procédé selon la revendication 7, dans lequel le choc électrique est appliqué 1 à 1000 fois, 10 à 900 fois, 20 à 800 fois, 30 à 700 fois, 40 à 600 fois, 50 à 500 fois, 60 à 400 fois, 70 à 300 fois, 80 à 200 fois ou 90 à 100 fois ; et/ou
la fréquence du choc électrique est de 1 à 1000 Hz, 10 à 900 Hz, 20 à 800 Hz, 30 à 700 Hz, 40 à 600 Hz, 50 à 500 Hz, 60 à 400 Hz, 70 à 300 Hz, 80 à 200 Hz ou 90 à 100 Hz.

12. Procédé selon l'une quelconque des revendications 7 à 11, dans lequel le champ électrique est appliqué par l'intermédiaire d'une cuvette d'électroporation.

13. Procédé selon l'une quelconque des revendications 7 à 11, dans lequel le champ électrique est appliqué par l'intermédiaire d'un film conducteur ; et
de préférence, le film conducteur est préparé à partir de l'acide poly-L-lactique (PLLA) et de nanotubes de carbone à parois multiples (WCNT) par coulée en mélange ou électrofilage.

14. Procédé selon la revendication 13, dans lequel le film conducteur est préparé par coulée en mélange, comprenant :
1) la dissolution de l'acide poly-L-lactique dans un solvant jusqu'à dissolution complète ;
2) la dispersion des nanotubes de carbone à parois multiples dans le même solvant ;
3) la réalisation d'une ultrasonication sur le mélange de nanotubes de carbone à parois multiples de l'étape 2), et l'ajout dans la solution d'acide poly-L-lactique de l'étape 1), pour obtenir une concentration finale d'acide poly-L-lactique entre 1 et 50 % p/v ;
4) après l'ultrasonication, la coulée du résultat sur une plaque et le maintien au repos ;
5) le décollement du film formé sur la plaque ; et
6) le placement du film dans un four pour évaporer complètement le solvant et obtenir un film conducteur.

15. Procédé selon la revendication 13, dans lequel l'acide poly-L-lactique présente un poids moléculaire compris dans une plage de 10 000 à 1 000 000 daltons et une viscosité intrinsèque de 1 à 20 dl/g.
